# EUROPEAN PATENT APPLICATION

(11) **EP 2 601 930 A1**
(43) Date of publication of application: **12.06.2013**
(21) Application number: 11814538.2
(22) Date of filing: 28.07.2011
(51) Int. Cl.: A61K 8/49, A61Q 19/02

(54) **AGENT FOR ENHANCING WHITENING EFFECT AND USE OF SAME**

(30) Priority: 03.08.2010 JP 2010174583
(71) Applicant: Hayashibara Co., Ltd., Okayama-shi, Okayama 702-8006 (JP)
(72) Inventor: MIYAKE Masaki, Okayama-shi Okayama 702-8006 (JP); SANO Osamu, Okayama-shi Okayama 702-8006 (JP); IWAKI Kanso, Okayama-shi Okayama 702-8006 (JP); OKURA Takanori, Okayama-shi Okayama 702-8006 (JP); FUKUDA Shigeharu, Okayama-shi Okayama 702-8006 (JP)
(74) Representative: Daniels, Jeffrey Nicholas
(86) International application number: PCT/JP2011/067264
(87) International publication number: WO 2012/017911

(57) **Abstract**

The present invention has objects to provide an agent for enhancing the effect of skin-whitening ingredients which enhances the skin-whitening action of skin-whitening ingredients and has an improved safeness, and to provide a skin-whitening agent, which contains the above agent and a skin-whitening ingredient(s) and has an improved and enhanced skin-whitening action. The present invention solves the above obj ects by providing an agent for enhancing the effect of skin-whitening ingredients, which contains one or more members selected from the group consisting of guanine and derivatives thereof as an effective ingredient(s); and a skin-whitening agent which contains the above agent along with a skin-whitening ingredient(s), particularly, one or more members selected from the group consisting of adenine including derivatives thereof and/or equol including derivatives thereof.

## Description

### Technical Field

The present invention relates to an agent for enhancing the effect of skin-whitening ingredients and uses thereof, particularly, an agent for enhancing the effect of skin-whitening ingredients containing guanine including derivatives thereof as an effective ingredient(s), and a skin-whitening agent containing such agent along with a skin-whitening ingredient (s), particularly, adenine including derivatives thereof, which has an improved and enhanced skin-whitening effect.

### Background Art

Although all of the induction mechanisms of spots, etc., in the skin have not been revealed, it is generally considered that spots in the skin are induced by abnormal intradermal deposition of melanin pigments formed by exposing the skin to the sunlight or ultraviolet rays. The melanin pigments, which may cause pigmentation in the skin, are formed in melanin-forming granules (or melanosomes) which are present in melanin cells (or melanocytes) existing between the epidermis and dermis, and diffused into neighboring cells.

It has been considered that the biochemical reaction of melanin formation in the melanocytes is the successive conversions of tyrosine into dopaquinone by the action of tyrosinase, and of dopaquinone into a blackmelanin through an enzymatic or non-enzymatic oxidation. As skin-whitening ingredients and compositions for whitening the skin based on the action mechanism of inhibiting the melanin formation, etc., various compounds including ascorbic acid, derivatives thereof, and compositions containing such have been conventionally proposed (see, for example, Japanese Patent Kokai Nos. 139288/91 and 135992/91), and also adenine and adenosine are examples of such (see, for example, Japanese Patent Kokai No. 186809/89 or International Patent Publication No. WO2003/84485). However, since a sufficient skin-whitening effect may not be obtained when the above ingredients are used alone, there has been also proposed a combination use thereof with other skin-whitening ingredient(s) to enhance the above effect (see, for example, Japanese Patent Kokai No. 2004-67576). Among compounds having a melanin-formation-inhibitory action, there has been known a compound such as azelaic acid that has been pointed out its skin stimulation, cytotoxicity, etc., in spite of its satisfactorily skin-whitening action (see, for example, Fragrance Journal, Extra Edition No. 14, pp. 11-24, 1995).

### Disclosure of Invention

### Object of the Invention

The present invention has objects to provide an agent forenhancingtheeffectofskin-whiteningingredients, whichenhances the skin-whitening action of skin-whitening ingredients and has an improved safeness; and a skin-whitening agent which contains the above agent and a skin-whitening ingredient (s) and has an improved and enhanced skin-whitening action.

The inventors of the present invention earnestly continued researching on compounds having a skin-whitening action by focusing on living-body-related compounds, particularly, nucleic-acids-related compounds interms of safeness, and they totally unexpectedly found that guanine including derivatives thereof per se have no skin-whitening action, but distinctly enhance the skin-whitening action of skin-whitening ingredients, particularly, adenine including derivatives thereof, and they can be used as agents for enhancing the effect of skin-whitening ingredients. Further, they accomplished the present invention by establishing a skin-whitening agent containing the above agent, which has guanine including derivatives thereof as an effective ingredient, and a skin-whitening ingredient(s), particularly, adenine including derivatives thereof, wherein the skin-whitening action of such a skin-whitening ingredient(s) is distinctly enhanced.

The present invention solves the above one object by providing an agent for enhancing the effect of skin-whitening ingredients, which contains one or more members selected from guanine including derivatives thereof as an effective ingredient(s).

The present invention further solves the above another object by providing a skin-whitening agent which contains both an agent for enhancing the effect of skin-whitening ingredients that contains one or more members selected from guanine including derivatives thereof as an effective ingredient and a skin-whitening ingredient (s), particularly, oneormoremembers selectedfromadenine including derivatives thereof.

The agent for enhancing the effect of skin-whitening ingredients, which contains guanine including derivatives thereof as an effective ingredient, of the present invention effectively enhances the skin-whitening action of skin-whitening ingredients, particularly, adenine including derivatives thereof and equol including derivatives thereof.

The skin-whitening agent of the present invention, which has an enhanced skin-whitening action of skin-whitening ingredients by the above agent of the present invention, has a satisfactory effect of paling or whitening pigmentation, spots, freckles, chloasma, etc., in the skin, which are accompanied by sunburn, inflammation, or aging.

### Best Mode for Carrying Out the Invention

As described above, the present invention relates to an agent for enhancing the effect of skin-whitening ingredients containing guanine including derivatives thereof as an effective ingredient, and a skin-whitening agent containing the above agent and a skin-whitening ingredient(s), particularly, adenine including derivatives thereof and/or equol including derivatives thereof in combination, which has an improved and enhanced skin-whitening effect.

It has never been reported that guanine including derivatives thereof enhance the skin-whitening action of skin-whitening ingredients, specifically, they enhance the skin-whitening action of adenine including derivatives thereof and equol including derivatives thereof, and this was firstly found by the inventors of the present invention.

In the present invention, the ratio of the agent for enhancing the effect of skin-whitening ingredients, which contains guanine including derivatives thereof as an effective ingredient, and a skin-whitening ingredient (s), particularly, adenine including derivatives thereof or equol including derivatives thereof is not specifically restricted, as long as the desired effect of the present invention is obtained. Usually, the molar ratio of guanine including derivatives thereof to adenine including derivatives thereof or equol including derivatives thereof is preferably 1:199 to 99:1, more preferably, 1:99 to 49:1. When the ratio of guanine including derivatives thereof used is lower than the above ratio, it may not exert the effect of enhancing the skin-whitening action of adenine including derivatives thereof and equol including derivatives thereof. On the contrary, when the ratio of guanine including derivatives thereof used exceeds the above ratio, it may not exert the effect of enhancing the skin-whitening action of adenine including derivatives thereof or equol including derivatives thereof that matches the ratio used.

Now explaining the agent for enhancing the effect of skin-whitening ingredients of the present invention, the term "guanine including derivatives thereof" used as an effective ingredient in the present invention means guanine, guanosine, guanosine monophosphate, guanosine diphosphate, guanosine triphosphate, and guanosine glycosides such as glucosylguanosine and salts thereof, which can be used singly or plurally in a mixture form. Any of those which are in the form of a keto- or enol-form or a mixture-form thereof can be used. In particular, among the guanine including derivatives thereof, a skin-whitening ingredient, particularly, guanine and glucosylguanosines are preferable because they have a relatively strong effect of enhancing the skin-whitening action of skin-whitening ingredients, particularly, adenine including derivatives thereof. Further, compounds such as guanine phosphates and glucosylguanosine having a relatively high solubility in aqueous solvents are preferable in terms of ease of handling or mixing with other base material ingredients for external dermatological agents, and particularly, glucosylguanosine is preferable.

As the agent for enhancing the effect of skin-whitening ingredients of the present invention, guanine including derivatives thereof as an effective ingredient can be used alone and optionally with other ingredient(s) in combination within the scope of the present invention. Examples of the other ingredients include one or more carriers, fillers/adjuvants/diluents/excipients/vehicles, stabilizers, buffers, pH-controlling agents, solvents, appropriate auxiliary agents, etc., which can be usually used for cosmetics, pharmaceuticals, or quasi-drugs according to the application and the form of the agent for enhancing the effect of skin-whitening ingredients of the present invention. In addition, the following optional medical ingredients can be also appropriately incorporated into the agent; antioxidants, humectants, licorice extracts, anti-inflammatories such as glycyrrhizinate and its derivatives and salts, vitamin P including derivatives thereof, and various crude drugs. In addition, sequestering agents such as edetate (EDTA) disodium, edetate (EDTA) trisodium, sodium citrate, sodium polyphosphate, sodiummetaphosphate, and gluconic acid; saccharides and sugar alcohols such as sucrose, trehalose, saccharide derivatives of trehalose, cyclictetrasaccharide, maltitol, and maltotriitol; and other biologically active ingredients and animal- and plant-extracts can be appropriately incorporated.

Varying depending on the types of guanine including derivatives thereof used, the content of guanine including derivatives thereof as an effective ingredient of the agent for enhancing the effect of skin-whitening ingredients of the present invention should not specifically be restricted as long as the content of guanine including derivatives thereof and the ratio of the content against adenine including derivatives thereof are within the range that attains the desiredeffect of the present invention, when incorporated into external dermatological agents. Too low content of guanine including derivatives thereof in the agent for enhancing the effect of skin-whitening ingredients is not preferable because, when incorporated into such external dermatological agents, the content of the agent in the external dermatological agents becomes too much to attain the desired effect of the present invention and may affect the physical property of the external dermatological agents.

Next explaining the skin-whitening agent of the present invention, the term "skin-whitening ingredient (s)" as the effective ingredient (s) in the skin-whitening agent of the present invention means a compound(s) or a substance(s) containing the same which is(are) safely applicable to the skin and has (have) a melanine-formation inhibitory action. Concretely, examples of such include adenine and derivatives thereof; L-ascorbic acid and its derivatives and salts such as magnesium L-ascorbic acid phosphate and L-ascorbic acid glucosides; alkoxysalicylic acid and salts thereof; equol including derivatives thereof (maybe called "equols", hereinafter); ellagic acid and salts thereof; glabridin; kojic acid and derivatives thereof; tocopherols; tranexamic acid and its derivatives and salts; tetrahydrocurcuminoid; hydroquinone glycosides and derivatives thereof; linoleic acid and salts thereof; resorcin and derivatives thereof; tranexamic acid and derivatives thereof; and plant- and animal-extracts such as indigo extracts, camomile extracts, and placenta extracts. Specifically among them, adenine including derivatives thereof andequols,which are relatively highly enhanced their skin-whitening action by guanine including derivatives thereof, are preferable, and particularly, adenine including derivatives thereof are preferable.

The term "adenine including derivatives thereof" used in the present invention means adenine, adenosine, adenosine monophosphate, adenosine diphosphate, adenosine triphosphate, adenosine glycosides such as glucosyladenosine and salts thereof, which can be used singly or plurally in a mixture form. They also include those in an amino-form, imino-form, or a mixture-form thereof. Specifically among these adenine including derivatives thereof, adenine and glucosyladenosine are preferable in terms of skin-whitening effect. In respect of ease of handling or mixing with other base material ingredients for external dermatological agents, compounds such as adenosine phosphates and glucosyladenosines which have a relatively-high solubility in aqueous solvents are preferable, and particularly, glucosyladenosine is preferable.

The term "equol including derivatives thereof" used in the present invention means equol and glycosides thereof, wherein the constituent equol can be either or both of two isomers, S- and R-isomers (hereinafter called "S-equol" and "R-equol", respectively, throughout the specification), or a mixture of two or more of these equol and glycosides thereof. In respect of the strength of skin-whitening action, the higher the content of S-equol and glycosides thereof to the total contents of equol and glycosides thereof, the more preferable it is, and 75% or more is more preferable.
In respect of ease of handling or mixing with other base material ingredients for external dermatological agents, equol glycosides having a relatively-high solubility in aqueous solvents are preferable.

Examples of the equol glycosides used in the present invention include, for example, six types of equol glycosides (may be called "glycosylequols", hereinafter) such as 4'-O-α-D-glycopyranosyl-equol, wherein a glycosyl group binds in an α-fashion to the 4'-OH group of S- and/or R-equols; 7-O-α-D-glycopyranosyl-equol, wherein a glycosyl group binds in an α-fashion to the 7-OH group of S- and/or R-equols; and 4',7-O-α-D-glycopyranosyl-equol, wherein a glycosyl group respectively binds in an α-fashion to the 4'- and 7-OH groups of S- and/or R-equols, as disclosed in International Patent Publication No. WO2008/126752. In glycosylequols,the number of glucose moieties as constituents of each glycosyl group should not specifically be restricted, however, the number is preferably one to six, more preferably, one to four, and most preferably, one in respect of ease of production or handling. As three forms of glycosylequols with different glycosyl-group-bonding-fashions exist in respective two equol isomers, any one of which or a mixture of at least two of which can be used, and they may contain equol used as a production material in practicing the present invention.

The skin-whitening agent of the present invention can be the one consisting of a skin-whitening ingredient(s) as the effective ingredient(s), particularly, adenine including derivatives thereof and/or equol including derivatives thereof along with guanine including derivatives thereof which enhance the skin-whitening action of the skin-whitening ingredient (s), and the agent may optionally contain other ingredient (s) without departing from the scope of the present invention. Examples of the other ingredients include any compounds and ingredients usable in cosmetics, pharmaceuticals, and quasi-drugs similar to the other ingredients which can be incorporated into the above-identified agent for enhancing the effect of skin-whitening ingredients, depending on the application and the form of the skin-whitening agent of the present invention.

The content of a skin-whitening ingredient(s), particularly, adenine including derivatives thereof and/or equol including derivatives thereof and guanine including derivatives thereof that enhances the skin-whitening action of adenine including derivatives thereof and/or equol including derivatives thereof is not specifically restricted as long as the desired effect of the present invention is exerted, varying depending on the types of adenine including derivatives thereof, equol including derivatives thereof, and guanine including derivatives thereof. Too low content of guanine including derivatives thereof and adenine including derivatives thereof and/or equol including derivatives thereof in the skin-whitening agent is not preferable when incorporated into an external dermatological agent, because the content of the skin-whitening agent in the external dermatological agent becomes too much to attain the desired effect of the present invention and may affect the physical property of the external dermatological agent.

The agent for enhancing the effect of skin-whitening ingredients of the present invention or the skin-whitening agent containing the above agent and adenine including derivatives thereof and/or equol including derivatives thereof of the present invention can be usually used to be incorporated into a base material ingredient (s) for external dermatological agents used in cosmetics, pharmaceuticals, or quasi-drugs. Concretely, base material ingredients generally used in cosmetics, pharmaceuticals, or quasi-drugs such as humectants, antioxidants, oily ingredients, ultraviolet absorbers, ultraviolet reflectors, surfactants, antibiotics, thickeners, alcohols, saccharides, sugar alcohols, powdered ingredients, coloring materials, flavors, aqueous ingredients, water, dermatological nutritional agents, animal- and plant-extracts, etc., can be arbitrarily incorporated. The form of the external dermatological agents incorporated with the agent for enhancing the effect of skin-whitening ingredients or the skin-whitening agent of the present invention includes any forms of ointments, creams, milky lotions, lotions, cosmetic lotions, gels, packs, bath salts, shampoos, rinses, dentifrices, etc., independently of the forms thereof.

The content of the agent for enhancing the effect of skin-whitening ingredients or the skin-whitening agent of the present invention in an external dermatological agent should not specifically be restricted as long as the content and the composition ratio are within the ranges that attain the desired skin-whitening effect of the present invention. Usually, the content of guanine including derivatives thereof is 0.0001 to 5%, preferably, 0.001 to 5%, more preferably, 0.01 to 2% in terms of guanine.

Incidentally, the guanine including derivatives thereof and skin-whitening ingredients such as adenine including derivatives thereof and equol including derivatives thereof can be obtained in a desired amount by conventional methods or in accordance therewith without any restriction of their origins and production methods, and even commercialized products can be arbitrarily used. Glycosides such as glucosylguanosine, glucosyladenosine, and glycosylequol can be prepared by using saccharide-transferring enzymes such as CGTase.

The present invention will be explained in detail based on comparative experiments on melanin-formation-inhibitory effect using nucleic-acid-related compounds.

### <Experiment 1: Influence of nucleic-acid-related compounds on melanin-formation inhibition>

The effect of nucleic-acid-related compounds on melanin-formation inhibition was evaluated by using mouse B16 melanoma cells frequently used as an index for evaluating skin-whitening agents used in external dermatological agents. Since nucleic-acid-related compounds are roughly classified into purine and pyrimidine compounds, the following respective representative compounds were used as test samples.

### <Test samples>

Purine compounds: Adenine, guanine, xanthine, hypoxanthine, and inosine, all of which are special-reagent-grade specimens commercialized by Sigma-Aldrich Co. LLC., St. Louis, MO, USA.
Pyrimidine compounds: Cytosine, thymine, and uracil, all of which are special-reagent-grade specimens commercialized by Sigma-Aldrich Co. LLC., St. Louis, MO, USA.
Positive control: Kojic acid, a special-reagent-grade specimen commercialized by Katayama Katayama Chemical, Inc., Tokyo, Japan.

### <Evaluation method>

Mouse B16 melanoma cells, which had been suspended in RPMI 1640 medium supplemented with 10% by volume of fetal calf serum (abbreviated as "FCS", hereinafter), were inoculated to "6-WELL MALTIWELL PLATE", a product name of a 6-well plate commercialized by Becton, Dickinson and Company, NJ, USA, in an amount of 2 x 10⁴ cells/4 ml/well and allowed to adhere to the bottom surface of each well. After removing the culture supernatant in each well adhered with the cells, RPMI 1640 medium supplemented with 10% by volume of FCS, to which had been added any one of the above test samples to give the concentration (10 µM) in the culture as shown in Table 1, was added to each well in an amount of 4 ml/well, followed by culturing the cells at 37°C for five days under the condition of 5% CO₂ by volume. Thereafter, cells were collected after adding trypsin-EDTA to each well and subjecting the resultant to centrifugation. The collected cells were washed once with Dulbecco's PBS (-) (abbreviated as "D-PBS", hereinafter) and centrifuged to remove the supernatant. To the remaining cell pellet was added 0.5 ml of 1N sodium hydroxide to solubilize the cells. The resulting alkali-solubilized solution was boiled for 30 min and determined for melanin content by the following method. As a negative control, a fresh preparation of the same medium as used in the above was cultured alone, while, as a positive control, mouse B16 melanoma cells were cultured for five days similarly as above after the addition of 4 ml/well of RPMI 1640 medium supplemented with 10% by volume of FCS, to which had been added kojic acid used as an effective ingredient for an external dermatological composition for skin-whitening to give a concentration of 500 µM. Each of these wells was assayed for melanin content similarly as above. When the cells were cultured in a culture medium supplemented with any of the test samples or kojic acid (a positive control), relative values of melanin content were calculated by regarding the melanin content of the negative control as 100, and the data are shown in Table 1 as melanin formation percentages (%). Any of the test samples (including kojic acid) used in this and the following experiments did not affect the proliferation of mouse B16 melanoma cells at the concentrations tested.

### <Assay for melanin content>

Each alkali-solubilized solution was measured for absorbance at a wavelength of 450 nm (650 nm as a reference) by using "M-Vmax", a product name of a microplate reader commercialized by Wako Pure Chemical Industries, Ltd., Tokyo, Japan, and comparing with that of a melanin standard specimen commercialized by Sigma-Aldrich Co. , LLC., St. Louis, MO, USA. In this and the following experiments, it means that the lower the melanin formation percentage, the higher the melanin-formation inhibitory effect, i.e., the skin-whitening effect.

**Table 1**

| Test sample | Concentration of test sample in liquid culture medium (µM) | Melanin formation percentage (%) |
|---|---|---|
| Culture medium alone (negative control) | 0 | 100 |
| Kojic acid (positive control) | 500 | 71 |
| Adenine | 10 | 60 |
| Guanine | 10 | 98 |
| Xanthine | 10 | 100 |
| Hypoxanthine | 10 | 97 |
| Inosine | 10 | 96 |
| Cytosine | 10 | 100 |
| Thymine | 10 | 97 |
| Uracil | 10 | 99 |

As evident from the results in Table 1, the melanin formation of mouse B16 melanoma cells was inhibited only when cultured with the addition of adenine at the concentrations used in the test. Whenever cultured by the addition of guanine, xanthine, hypoxanthine, inosine, cytosine, thymine, and uracil, no melanin-formation inhibition was observed. The result indicates that, among the nucleic-acid-related compounds tested, only adenine has a melanin-formation-inhibitory action and is effective as a skin-whitening ingredient.

### < Experiment 2: Test on melanin-formation-inhibitory effect when nucleic-acid-related compounds are used in combination>

A test for melanin-formation-inhibitory effect was conducted when adenine, which had only showed a melanin-formation inhibitory action in Experiment 1, was used in combination with other nucleic-acid-related compounds. Except for culturing the cells in a culture medium with the addition of any one or more of the test samples used in Experiment 1 as the above test samples to give the respective concentrations as shown in Table 2, melanin-formation percentages were determined by the same evaluation method as in Experiment 1, and the results are in Table 2.

**Table 2**

| Test sample | Concentration of test sample in culture medium (µM) | Melanin formation percentage (%) |
|---|---|---|
| Culture medium alone | 0 | 100 |
| Kojic acid | 500 | 72 |
| Adenine | 5 | 83 |
| Guanine | 5 | 98 |
| Xanthine | 5 | 100 |
| Hypoxanthine | 5 | 97 |
| Inosine | 5 | 98 |
| Cytosine | 5 | 98 |
| Thymine | 5 | 99 |
| Uracil | 5 | 98 |
| Adenine | 10 | 60 |
| Adenine | 5 | 32 |
| + | + | |
| Guanine | 5 | |
| Adenine | 5 | 80 |
| + | + | |
| Xanthine | 5 | |
| Adenine | 5 | 78 |
| + | + | |
| Hypoxanthine | 5 | |
| Adenine | 5 | 80 |
| + | + | |
| Inosine | 5 | |
| Adenine | 5 | 83 |
| + | + | |
| Cytosine | 5 | |
| Adenine | 5 | 85 |
| + | + | |
| Thymine | 5 | |
| Adenine | 5 | 85 |
| + | + | |
| Uracil | 5 | |

As evident from the result in Table 2, the melanin formation percentage was 83% when the cells were cultured by the addition of 5 µM adenine, while it was 60% when the cells were cultured by the addition of 10 µM adenine. No melanin-formation inhibition was found when the cells were cultured by the addition of 5 µM of respective guanine, xanthine, hypoxanthine, inosine, cytosine, thymine, and uracil other than adenine. In the case of using adenine along with other test sample, a distinct melanin-formation inhibition was observed as a melanin formation percentage of 32% when adenine and guanine were used in respective amounts of 5 µM. No significant difference in melanin-formation-inhibitory percentage was observed between the case with the addition of adenine alone and the case with the addition of adenine along with xanthine, hypoxanthine, inosine, cytosine, thymine, or uracil. Based on these results, it was revealed that the skin-whitening action of adenine is unexpectedly, distinctly enhanced only when used in combination with guanine among nucleic-acid-related compounds with no melanin-formation-inhibitory action.

### <Experiment 3: Test on melanin-formation-inhibitory effect of adenine including derivatives thereof and guanine including derivatives thereof>

Since adenine was observed to have a relatively strong melanin-formation-inhibitory action in Experiment 1, the melanin-formation-inhibitory strengths of adenine including derivatives thereof were compared in accordance with the method in Experiment 1. Also, the melanin-formation-inhibitory action of guanine including derivatives thereof, which enhance the melanin-formation-inhibitory action of adenine, were reconfirmed. By using the following test samples as adenine including derivatives thereof and guanine including derivatives thereof, each compound was added to a culture medium to give the respective concentrations in Table 3 or 4 and determined for melanin formation percentage by the same evaluation method as in Experiment 1. The results for adenine including derivatives thereof and guanine including derivatives thereof are respectively in Tables 3 and 4.

### <Test samples>

Adenine including derivatives thereof: Adenine, adenosine, adenosine 5'-monophosphate, adenosine 5'-diphosphate, and adenosine 5'-triphosphate (all of which are special-reagent-grade specimens commercialized by Sigma-Aldrich Co. LLC., St. Louis, MO, USA.), and glucosyladenosine;
Guanine including derivatives thereof: Guanine, guanosine, guanosine 5'-monophosphate, guanosine 5'-diphosphate, and guanosine 5'-triuphosphate (all of which are special-reagent-grade specimens commercialized by Sigma-Aldrich Co. LLC., St. Louis, MO, USA.), and glucosylguanosine;
Positive control: Kojic acid (a special-reagent-grade specimen commercialized by Katayama Chemical Inc., Tokyo, Japan)
The glucosyladenosine and glucosylguanosine were respectively prepared by the following methods at Hayashibara Biochemical Laboratories, Inc., Okayama, Japan.

### <Preparation of glucosyladenosine>

Adenosine (a special-reagent-grade specimen commercialzied by Tokyo Chemical Industry Co., Ltd., Tokyo, Japan) and dextrin ("PINEDEX #1", a solid content of about 92.3%, a product name of a dextrin commercialized by Matsutani Chemical Industry Co., Ltd., Hyogo, Japan) were added to 10 mM sodium acetate solution (pH 5.5) to give respective concentrations of 1% and 10%, followed by heating the resulting solution at 50°C to completely dissolve the contents while stirring. A CGTase, which had been prepared from *Geobacillus stearothermophilus* Tc-91 stain (deposited with International Patent Organism Depositary in National Institute of Advanced Industrial Science and Technology, Tsukuba Central 6, 1-1, Higashi 1-chome, Tsukuba-shi, Ibaraki-ken, 305-8566 Japan, under the accession number of FERM P-2225 and transferred to an international deposition under the accession number of FERM BP-11273 on September 30, 2010), was added to the above solution in an amount of 1,000 units/g dextrin, and subjected to an enzymatic reaction at 50°C for 24 hours, heated at 100°C for 15 min to inactivate the remaining CGTase, admixed with "GLUCOZYME #20000", a product name of a glucoamylase specimen commercialized by Nagase ChemteX Corp., Osaka, Japan, in an amount of 260 units/g dextrin, and subjected to an enzymatic reaction at 50°C for 24 hours. The resulting reaction solution was heated at 100°C for 10 min and centrifuged at 11,500 rpm to collect an 800 ml supernatant. The supernatant was fed to an activated charcoal column (120 mm x ø41 mm) with a volume of 150 ml at a flow rate of SV 3 (5 ml/min) to adsorb glucosyladenosine and adenosine thereupon, washed with deionized water in a volume of 7-times of the column volume and 20% ethanol solution in a volume of 6-times of the column volume, and eluted with 40% ethanol solution. The eluate was fractionated by 50 ml aliquots, followed by collecting fractions observed with an ultraviolet absorption at a wavelength of 260 nm. The collected fractions were pooled, filtered with a membrane filter having a pour size of 0.22 µm, and subjected to a fractional HPLC using an ODS column to collect fractions with glucosyladenosine. The collected fractions were pooled, desalted with a column chromatography using an activated charcoal, and eluted with 40% ethanol solution to collect fractions with glucosyladenosine. The collected fractions were pooled, filtered with a membrane filter having a pore size of 0.22 µm, and dried *in vacuo* to obtain a powdered glucosyladenosine with a purity of at least 98%, on a dry solid basis (d.s.b.).

### <Preparation of glucosylguanosine>

A glucosylguanosine with a purity of at least 98%, d.s.b, was prepared by the same method as the above preparation method for glucosyladenosine except for replacing the adenosine with guanosine (a special-reagent-grade specimen commercialized by Tokyo Chemical Industry Co., Ltd., Tokyo, Japan).

**Table 3**

| Test sample | Concentration of test sample in liquid culture medium (µM) | Melanin formation percentage (%) |
|---|---|---|
| Culture medium alone | 0 | 100 |
| Kojic acid | 500 | 72 |
| | 1000 | 49 |
| | 2000 | 30 |
| Adenine | 5 | 83 |
| | 10 | 60 |
| | 20 | 39 |
| Adenosine | 5 | 94 |
| | 10 | 71 |
| | 20 | 48 |
| Adenosine 5'-monophosphate | 5 | 100 |
| | 10 | 82 |
| | 20 | 60 |
| Adenosine 5'-diphosphate | 5 | 93 |
| | 10 | 81 |
| | 20 | 51 |
| Adenosine 5'-triphosphate | 5 | 93 |
| | 10 | 80 |
| | 20 | 53 |
| Glucosyladenosine | 5 | 89 |
| | 10 | 63 |
| | 20 | 43 |

**Table 4**

| Test sample | Concentration of test sample in liquid culture medium (µM) | Melanin formation percentage (%) |
|---|---|---|
| Culture medium alone | 0 | 100 |
| Kojic acid | 500 | 72 |
| | 1000 | 49 |
| | 2000 | 30 |
| Guanine | 5 | 98 |
| | 10 | 98 |
| | 20 | 101 |
| Guanosine | 5 | 98 |
| | 10 | 102 |
| | 20 | 97 |
| Guanosine 5'-monophosphate | 5 | 100 |
| | 10 | 101 |
| | 20 | 101 |
| Guanosine 5'-diphosphate | 5 | 93 |
| | 10 | 100 |
| | 20 | 95 |
| Guanosine 5'-triphosphate | 5 | 95 |
| | 10 | 98 |
| | 20 | 97 |
| Glucosylguanosine | 5 | 98 |
| | 10 | 99 |
| | 20 | 99 |

As shown in Table 3, kojic acid (a positive control) inhibited the melanin formation by mouse B16 melanoma cells in a concentration dependent manner. In the range tested, any of adenosine including derivatives thereof significantly inhibited the melanin formation by mouse B16 melanoma cells in a concentration dependent manner similarly as in kojic acid. Comparing the melanin formation percentage when the cells were cultured in the presence of 10 µM adenosine including derivatives thereof, adenine, adenosine, and glucosyladenosine showed a stronger melanin-formation-inhibitory effect than those of adenosine phosphates; and adenine and glucosyladenosine gave the strongest results. On the contrary, as shown in Table 4, the melanin formation percentages of any of guanine including derivatives thereof at the concentrations used in this experiment did not give a significant difference compared to the negative control cultured with the culture medium alone, revealing that guanine including derivatives thereof *per* se are judged to have no melanin-formation-inhibitory action.

### <Experiment 4: Influence of guanine including derivatives thereof on theskin-whitening action of adenine including derivatives thereof>

Since guanine was confirmed to enhance the skin-whitening action of adenine in Experiment 2, a test for examining the influence of the addition of guanine including derivatives thereof on the skin-whitening action of adenine including derivatives thereof was conducted in accordance with the method in Experiment 2 and the results were evaluated by using the same method as in Experiment 1. The melanin formation percentages were determined by using any of the same test samples as in Experiment 3, adding the combinational compounds of the test samples in Tables 5 to 7 to a culture liquid to give the concentrations in the tables, and culturing mouse B16 melanoma cells therein. The results are in Tables 5 to 7.

**Table 5**

| Test sample | Concentration of test sample in liquid culture medium (µM) | Melanin formation percentage (%) |
|---|---|---|
| Culture medium alone | 0 | 100 |
| Kojic acid | 2000 | 30 |
| Adenine | 2.5 | 95 |
| | 5 | 83 |
| Guanine | 2.5 | 99 |
| | 5 | 98 |
| Guanosine | 2.5 | 98 |
| | 5 | 98 |
| Guanosine 5'-monophosphate | 2.5 | 99 |
| | 5 | 100 |
| Glucosylguanosine | 2.5 | 98 |
| | 5 | 98 |
| Adenine + Guanine | 2.5 | 44 |
| | + | |
| | 2.5 | |
| | 5 | 32 |
| | + | |
| | 5 | |
| Adenine + Guanosine | 2.5 | 65 |
| | + | |
| | 2.5 | |
| | 5 | 48 |
| | + | |
| | 5 | |
| Adenine + Guanosine 5'-monophosphate | 2.5 | 61 |
| | + | |
| | 2.5 | |
| | 5 | 48 |
| | + | |
| | 5 | |
| Adenine + Glucosylguanosine | 2.5 | 59 |
| | + | |
| | 2.5 | |
| | 5 | 44 |
| | + | |
| | 5 | |

**Table 6**

| Test sample | Concentration of test sample in liquid culture medium (µM) | Melanin formation percentage (%) |
|---|---|---|
| Culture medium alone | 0 | 100 |
| Kojic acid | 2000 | 30 |
| Adenosine | 2.5 | 98 |
| | 5 | 94 |
| Guanine | 2.5 | 99 |
| | 5 | 98 |
| Guanosine | 2.5 | 98 |
| | 5 | 98 |
| Guanosine 5'-monophosphate | 2.5 | 99 |
| | 5 | 100 |
| Glucosylguanosine | 2.5 | 98 |
| | 5 | 98 |
| Adenosine + Guanine | 2.5 | 59 |
| | + | |
| | 2.5 | |
| | 5 | 47 |
| | + | |
| | 5 | |
| Adenosine + Guanosine | 2.5 | 65 |
| | + | |
| | 2.5 | |
| | 5 | 52 |
| | + | |
| | 5 | |
| Adenosine + Guanosine 5'-monophosphate | 2.5 | 60 |
| | + | |
| | 2.5 | |
| | 5 | 51 |
| | + | |
| | 5 | |
| Adenosine + Glucosylguanosine | 2.5 | 61 |
| | + | |
| | 2.5 | |
| | 5 | 44 |
| | + | |
| | 5 | |

**Table 7**

| Test sample | Concentration of test sample in liquid culture medium (µM) | Melanin formation percentage (%) |
|---|---|---|
| Culture medium alone | 0 | 100 |
| Kojic acid | 2000 | 30 |
| Adenosine 5'-monophosphate | 2.5 | 97 |
| | 5 | 100 |
| Guanine | 2.5 | 99 |
| | 5 | 98 |
| Guanosine | 2.5 | 98 |
| | 5 | 98 |
| Guanosine 5'-monophosphate | 2.5 | 99 |
| | 5 | 100 |
| Glucosylguanosine | 2.5 | 98 |
| | 5 | 98 |
| Adenosine 5'-monophosphate + Guanine | 2.5 | 61 |
| | + | |
| | 2.5 | |
| | 5 | 48 |
| | + | |
| | 5 | |
| Adenosine 5'-monophosphate + Guanosine | 2.5 | 66 |
| | + | |
| | 2.5 | |
| | 5 | 51 |
| | + | |
| | 5 | |
| Adenosine 5'-monophosphate + Guanosine 5'-monophosphate | 2.5 | 63 |
| | + | |
| | 2.5 | |
| | 5 | 50 |
| | + | |
| | 5 | |
| Adenosine 5'-monophosphate + Glucosylguanosine | 2.5 | 60 |
| | + | |
| | 2.5 | |
| | 5 | 45 |
| | + | |
| | 5 | |

As shown in Tables 5 to 7, it was observed that none of guanine including derivatives thereof *per* se showed any melanin-formation-inhibitory action but they enhanced the action when coexisted with adenine including derivatives thereof as skin-whitening ingredients. The strengths of melanin-formation-inhibitory action of adenine including derivatives thereof by guanine including derivatives thereof were substantially the same among guanine, guanosine, guanosine 5'-monophosphate, and glucosylguanosine used in the test. The result indicates that guanine including derivatives thereof are useful as agents for enhancing the effect of skin-whitening ingredients for adenine including derivatives thereof as skin-whitening ingredients. It also indicates that the coexistence of guanine including derivatives thereof as agents for enhancing the effect of skin-whitening ingredients and adenine including derivatives thereof as skin-whitening ingredients can provide a skin-whitening agent with a satisfactory melanin-formation-inhibitory effect.

### <Experiment 5: Influence of the composition ratio of guanine including derivatives thereof and adenine including derivatives thereof on the skin-whitening action of adenine including derivatives thereof>

Since the melanin-formation-inhibitory action of adenine including derivatives thereof was confirmed to be enhanced by guanine including derivatives thereof in Experiments 2 and 4, a test for confirming the composition ratios of guanine including derivatives thereof and adenine including derivatives thereof that attain such enhancement effect was conducted by the same evaluation method as in Experiment 1 in accordance with the method in Experiment 2. The melanin formation percentage of mouse B16 melanoma cells was determined by mixing guanine and adenine in the molar ratios as shown in Table 8 and adding guanine and adenine to a culture medium to give a total concentration of 5 µM. The result is in Table 8.

**Table 8**

| Composition ratio of test samples (molar ratio) | | Melanin formation percentage (%) |
|---|---|---|
| Guanine | Adenine | |
| 0 | 0 | 100 |
| 0 | 1 | 83 |
| 1 | 0 | 98 |
| 1 | 999 | 83 |
| 1 | 199 | 75 |
| 1 | 99 | 63 |
| 1 | 49 | 55 |
| 1 | 9 | 48 |
| 3 | 7 | 45 |
| 1 | 1 | 44 |
| 7 | 3 | 48 |
| 9 | 1 | 58 |
| 49 | 1 | 66 |
| 99 | 1 | 72 |
| 199 | 1 | 92 |
| 999 | 1 | 97 |

As shown in Table 8, guanine enhanced the melanin-formation-inhibitory action of adenine in the range of 1:199 to 99:1 as a molar ratio of guanine and adenine, and the inhibitory action became distinct in the range of 1:99 to 49:1, and it became particularly distinct in the range of 1:9 to 7:3.

### <Experiment 6: Influence of guanine including derivatives thereof on the skin-whitening action of equols>

Since guanine including derivatives thereof were confirmed to enhance the skin-whitening action of adenine including derivatives thereof in Experiments 1 to 5, the influence of guanine including derivatives thereof on skin-whitening ingredients other than adenine including derivatives thereof was examined by using equol. In the test, equol andglycosylequols preparedbythe following method were used.

### <Preparation of glycosylequols>

In accordance with the method disclosed in Example 1 of International Patent Publication No. WO2008/126752, one gram of a reagent grade equol (Product Code: "26355-54", a mixture of R- and S-equols, commercialized by Nacalai Tesque, Inc., Kyoto, Japan) was dissolved in 100 ml ethanol, admixed with 100 g of "PINEDEX #1", a dextrin commercialized by Sanwa Starch Co., Ltd., Nara, Japan, and 1,000 ml of a solution containing 50 mM acetate buffer (pH 6.0) and 2 mM calcium chloride, admixed with 1,000 units/g solid of a CGTase derived from *Bacillus stearothermophilus* Tc-91 strain (FERM BP-11273) commercialzied by Hayashibara Biochemical Laboratories Inc., Okayama, Japan, and incubated at 40°C for 72 hours. The resultingmixture was heated at 100 °C to suspend the enzymatic reaction, admixed with 70 ml of 1M acetate buffer and 10 units/g solid of a glucoamylase specimen commercialized by Seikagaku Corporation, Tokyo, Japan, and incubated at 40°C for 18 hours. The resulting enzymatic reaction solution was fed to a column (3 cm ø x 90 cm, 640 ml) packed with "DIAION HP-20 Column", a product name of a macroporous synthetic absorbent commercialized by Mitsubishi Kasei Corp., Tokyo, Japan, followed by washing the column with water to remove unabsorbed saccharides, subsequently feeding 30% and 40% ethanol solutions to the column to collect a fraction containing glycosylequols eluted with 40% ethanol solution. By using an evaporator, ethanol in the fraction was removed to concentrate it, followed by collecting a fraction containing glycosylequols and freeze-drying the fraction to obtain a powder containing equol glycosides (a mixture of S- and R-equol-glycosides and is called "glucosylequols" hereinafter).

In accordance with the method in Experiment 1 of International Patent Publication No. WO2008/126752 and based on the disclosure of Example 1 in Tokuhyo No. 2006-504409, two grams of a commercialized equol (a mixture of S- and R-equols commercialized by Funakoshi Co., Ltd., Tokyo, Japan) was subjected to a column chromatography using "CHIRAL CELL OJ-H", 4.6 mm ø x 250 mm, a product name of a chiral column for separating optical isomers, commercialized by Daicel Corporation, Osaka, Japan, and using solution A (hexane: ethanol = 9:1) and solution B(hexane:ethanol = 1:9), wherein the feeding volume of solution B was 1 ml/min at a linear gradient increasing from 0% to 100% per 15 min, to separate and obtain Sand R-equols. The obtained 0.7 g of S-equol and 0.7 g of R-equol were respectively dissolved in 70 ml ethanol, admixed with 700 ml of a solution containing 70 g of "PINEDEX #1", a product name of a dextrin of Matsutani Chemical Industry Co., Ltd., Hyogo, Japan, 50 mM acetate buffer (pH 6.0), and 2 mM calcium chloride, admixed with 1,000 units/g solid of a CGTase derived from *Bacillus stearothermophilus* Tc-91 strain (FERM BP-11273) produced by Hayashibara Biochemical Laboratories, Inc., Okayama, Japan, and incubated at 40°C for 72 hours to obtain saccharide-transferring reaction solutions. The enzymatic reaction solutions were respectively heated at 100°C to suspend the enzymatic reaction, admixed with 70 ml of 1 M acetate buffer and 10 units/g solid of a glucoamylase specimen commercialized by Seikagaku Corporation, Tokyo, Japan, and incubated at 40°C for 18 hours. The resulting enzymatic reaction solutions were respectively fed to a column (30 mm ø x 900 mm, 640 ml) packed with "DIAION HP-20 Column", a product name of a macroporous synthetic absorbent commercialized by Mitsubishi Kasei Corp., Tokyo, Japan, followed by washing the column with water to remove unadsorbed saccharides, and sequentially feeding 10%, 20%, 30%, 40%, 50%, 60% and 100% ethanol solutions to the column to collect a fraction containing equol glycosides eluted with 40% ethanol solution. By using an evaporator, ethanol in the fraction was removed to concentrate it, followed by freeze-drying the resulting concentrate to obtain a powder containing S-equol glycosides (called "glucosyl-S-equols", hereinafter) and a powder containing R-equol glycosides (called "glucosyl-R-equols", hereinafter).

The above powder containing glucosyl-S-equols was redissolved, fed to a fractional high-performance liquid chromatography using D-ODS-5 column (may be abbreviated as "HPLC", hereinafter) under the following conditions, eluting with acetonitrile/water/acetic acid (18:82:0.1) while monitoring the ultraviolet (may be abbreviated as "UV", hereinafter) absorption to collect an elution fraction with 7-O-α-D-glycopyranosyl-S-equol (called "7-glucosyl-S-equol", hereinafter) and an elution fraction with 4'-O-α-D-glycopyranosyl-S-equol (called "4'-glucosyl-S-equol", hereinafter), which were then subjected to an evaporator to remove solvent, concentrated, and freeze-dried.

### <HPLC: For separation>

Apparatus: CR-4A and LCD-10AD, commercialized by Shimadzu Corporation, Kyoto, Japan
Column: D-ODS-5 S-5 20 mm ø x 250 mm, commercialized by YMC Co., Ltd., Kyoto, Japan
Moving phase: Acetonitrile/water/acetic acid (18:82:0.1)
Flow rate: 6 ml/min
Temperature: 40°C
Detection: UV

The contents of glucosylequol in each glucosylequol prepared in the above was determined based on both the elution peak area for each ingredient in the chart of HPLC elution pattern for UV 280 nm absorbance by the following fractional HPLC and the molar adsorption coefficient at a wavelength of UV 280 nm (equol and glucosylequol were calculated by regarding them as having the same molar adsorption coefficient at a wavelength of UV 280 nm because a saccharide moiety in a glycoside has no ultraviolet absorption).

### <HPLC: For analysis>

Apparatus: CR-4A and LCD-10AD, commercialized by Shimadzu Corporation, Kyoto, Japan
Column: ODS-AM-303 4.6 mm ø x 250 mm, commercialized by YMC Co., Ltd., Kyoto, Japan
Moving phase: Solution A : Acetonitrile/water/acetic acid (20:80:0.1)
   Solution B: Acetonitrile/water/acetic acid (40:60:0.1)
Flow rate: 0.8 ml/min
   It was conducted under a time schedule of successively eluting with solution A for 25 min, with solution B while increasing the concentration from 0% to 100% over 10 min, and with solution B for 10 min.
Temperature: 35°C
Detection: UV
   The UV absorbance in the above fractional HPLC and analytical HPLC were monitored as follows:
   <UV>
Apparatus: SPD-10AVP commercialized by Shimadzu Corporation, Kyoto, Japan
Wavelength: UV 280 nm

### <Test method>

The melanin-formation percentage was determined by the same evaluation method as in Experiment 1 except for culturing the cells in a liquid culture medium supplemented with any one of the following ingredients alone or combination with guanine to give the concentrations as shown in Tables 9 and 10; equol (Product Code: "26355-54", a mixture of R- and S-equols, commercialized by Nacalai Tesque, Inc., Kyoto, Japan); and R-equol, S-equol, glucosylequol, glucosyl-S-equol, glucosyl-R-equol, 7-glucosyl-S-equol, and 4'-glucosyl-S-equol, which had been prepared in the above. The results are in Tables 9 and 10.

**Table 9**

| Test sample | Concentration of test sample in liquid culture medium (µM) | Melanin formation percentage (%) |
|---|---|---|
| Culture medium alone | 0 | 100 |
| Kojic acid | 2000 | 27 |
| Equol | 1 | 91 |
| | 5 | 67 |
| S-Equol | 1 | 86 |
| | 5 | 60 |
| R-Equol | 1 | 94 |
| | 5 | 77 |
| Glucosyl-S-equol | 1 | 87 |
| | 5 | 75 |
| Glucosyl-R-equol | 1 | 92 |
| | 5 | 82 |
| 4'-Glucosyl-S-equol | 1 | 85 |
| | 5 | 74 |
| 7-Glucosyl-S-equol | 1 | 85 |
| | 5 | 75 |
| Guanine | 1 | 100 |
| | 5 | 99 |

**Table 10**

| Test sample | Concentration of test sample in liquid culture medium (µM) | Melanin formation percentage (%) |
|---|---|---|
| Equol | 1 | 62 |
| + | + | |
| Guanine | 1 | |
| S-Equol | 1 | 53 |
| + | + | |
| Guanine | 1 | |
| R-Equol | 1 | 70 |
| + | + | |
| Guanine | 1 | |
| Glucosyl-S-equol | 1 | 62 |
| + | + | |
| Guanine | 1 | |
| Glucosyl-R-equol | 1 | 77 |
| + | + | |
| Guanine | 1 | |
| 4'-Glucosyl-S-equol | 1 | 61 |
| + | + | |
| Guanine | 1 | |
| 7-Glucosyl-S-equol | 1 | 62 |
| + | + | |
| Guanine | 1 | |

As shown in Tables 9 and 10, any of the equols used in this test was enhanced its skin-whitening action when used in combination with guanine similarly as in adenine including derivatives thereof. In terms of the strength of the inhibitory action against melanin formation, equol was stronger than its glycosides, and S-equols was tended to be stronger than R-equols. There was no difference in melanin formation level between 7-glucosyl-S-equol and 4'-glucosyl-S-equol and there was no difference in skin-whitening action inherent to the difference of the bonding site of glucose. Although concrete data are not shown, except for guanine including derivatives thereof, no enhancement effect of skin-whitening action of equols was observed among the nucleic acids used in Experiment 2. On that point, the same result was obtained as in adenine including derivatives thereof.

### <Experiment 7: Influence of guanine including derivatives thereof on the skin-whitening action of equols>

Since guanine was confirmed to enhance the skin-whitening action of equols in Experiment 6, a test for examining the influence of guanine including derivatives thereof on the skin-whitening action of equols was conducted by the same evaluation method as in Experiment 1 in accordance with the method in Experiment 2. The melanin formation percentage of mouse B16 melanoma cells was determined by culturing the cells in a culture liquid medium to which had been added equol in Table 11 or a combination of glucosylequol and guanine including derivatives thereof in Table 12 to give the concentrations in Tables 11 and 12. The results are in Tables 11 and 12.

**Table 11**

| Test sample | Concentration of test sample in liquid culture medium (µM) | Melanin formation percentage |
|---|---|---|
| Culture medium alone | 0 | 100 |
| Kojic acid | 2000 | 28 |
| Equol | 1 | 91 |
| | 5 | 65 |
| Guanine | 1 | 99 |
| | 5 | 99 |
| Guanosine | 1 | 99 |
| | 5 | 98 |
| Guanosine 5'-monophosphate | 1 | 99 |
| | 5 | 100 |
| Glucosylguanosine | 1 | 99 |
| | 5 | 98 |
| Equol + Guanine | 1 | 63 |
| | + | |
| | 1 | |
| | 5 | 48 |
| | + | |
| | 5 | |
| Equol + Guanosine | 1 | 66 |
| | + | |
| | 1 | |
| | 5 | 46 |
| | + | |
| | 5 | |
| Equol + Guanosine 5'-monophosphate | 1 | 65 |
| | + | |
| | 1 | |
| | 5 | 44 |
| | + | |
| | 5 | |
| Equol + Glucosylguanosine | 1 | 67 |
| | + | |
| | 1 | |
| | 5 | 45 |
| | + | |
| | 5 | |

**Table 12**

| Test sample | Concentration of test sample in liquid culture medium (µM) | Melanin formation percentage (%) |
|---|---|---|
| Culture medium alone | 0 | 100 |
| Kojic acid | 2000 | 28 |
| Glucosylequol | 1 | 91 |
| | 5 | 75 |
| Guanine | 1 | 99 |
| | 5 | 99 |
| Guanosine | 1 | 99 |
| | 5 | 98 |
| Guanosine 5'-monophosphate | 1 | 99 |
| | 5 | 100 |
| Glucosylguanosine | 1 | 99 |
| | 5 | 98 |
| Glucosylequol + Guanine | 1 | 73 |
| | + | |
| | 1 | |
| | 5 | 55 |
| | + | |
| | 5 | |
| Glucosylequol + Guanosine | 1 | 72 |
| | + | |
| | 1 | |
| | 5 | 50 |
| | + | |
| | 5 | |
| Glucosylequol + Guanosine 5'-monophosphate | 1 | 73 |
| | + | |
| | 1 | |
| | 5 | 51 |
| | + | |
| | 5 | |
| Glucosylequol + Glucosylguanosine | 1 | 76 |
| | + | |
| | 1 | |
| | 5 | 55 |
| | + | |
| | 5 | |

As shown in Tables 11 and 12, both of equol and glucosylequol were enhanced their skin-whitening actions by guanine including derivatives thereof used in the test. Since the equol used in the test consisted of S- and R-equols and the glucosylequol consisted of glucosyl-S-equol and glucosyl-R-equol, it can be concluded that equols are enhanced their skin-whitening action by guanine including derivatives thereof similarly as adenine including derivatives thereof when considering both the results in this experiment and Experiment 6. The results in these Experiments 6 and 7 indicate that guanine including derivatives thereof are useful as agents for enhancing the effect of skin-whitening ingredients for equols as skin-whitening ingredients. Also, these results indicate that a skin-whitening agent with a satisfactory melanin-formation-inhibitory effect can be made by coexisting both guanine including derivatives thereof as agents for enhancing the effect of skin-whitening ingredients and equols as skin-whitening ingredients.

### <Experiment 8: Influence of the composition ratio of guanine including derivatives thereof and equols on the skin-whitening action of equols>

Since it was confirmed that the melanin-formation-inhibitory action of equols are enhanced by guanine including derivatives thereof in Experiments 6 and 7, a test for confirming the composition ratios of guanine including derivatives thereof and equols that attain such enhancement effect was conducted by using the same evaluation method as in Experiment 1 in accordance with the method in Experiment 5. The melanin formation percentage of mouse B16 melanoma cells was determined by mixing guanine and equol in the molar ratios as shown in Table 13 and adding these guanine and equol to a culture medium to give a total concentration of 5 µM. The result is in Table 13.

**Table 13**

| Composition ratio of test samples (molar ratio) | | Melanin formation percentage (%) |
|---|---|---|
| Guanine | Equol | |
| 0 | 0 | 100 |
| 0 | 1 | 91 |
| 1 | 0 | 98 |
| 1 | 999 | 90 |
| 1 | 199 | 80 |
| 1 | 99 | 74 |
| 1 | 49 | 62 |
| 1 | 9 | 55 |
| 3 | 7 | 54 |
| 1 | 1 | 50 |
| 7 | 3 | 52 |
| 9 | 1 | 66 |
| 49 | 1 | 73 |
| 99 | 1 | 81 |
| 199 | 1 | 92 |
| 999 | 1 | 97 |

As shown in Table 13, guanine enhanced the melanin-formation-inhibitory action of equol in the range of 1:199 to 99:1 as a molar ratio of guanine and equol, and the inhibitory action became significant in the range of 1: 99 to 49:1, and it became particularly distinct in the range of 1:9 to 7:3.

### <Experiment 9: Influence of guanine including derivatives thereof and adenine including derivatives thereof on the melanin formation in the skin>

Since guanine including derivatives thereof was confirmed to enhance the melanin-formation-inhibitory action (or the skin-whitening action) of adenine including derivatives thereof in Experiments 2, 4 and 5, the melanin-formation-inhibitory action in the skin by suntan (or ultraviolet ray irradiation) was evaluated with 10 volunteers by using guanine (a special-reagent-grade specimen commercialized by Sigma-Aldrich Co. LLC., St. Louis, MO, USA) as guanine including derivatives thereof, and adenine (a special-reagent-grade specimen commercialized by Sigma-Aldrich Co. LLC., St. Louis, MO, USA) as adenine including derivatives thereof.

### <Subjects>

Ten males and females (five each), 22 to 55 years old

### <Ultraviolet ray irradiation apparatus>

Light source: "NS-8F MODEL" commercialized by Sanwa Medical Co., Ltd., Okayama, Japan
Ultraviolet ray fluorescent lamp: "FL-20SE", five pieces, commercialized by Toshiba Corporation, Tokyo, Japan

### <Measurement for minimum erythema dose>

At a dose of 25, 50, 75, 100, 125 or 150 mJ/cm², ultraviolet rays were irradiated to a part, which had been previously confirmed to have neither spot nor wound in the inside of the right upper arm of each volunteer, in the range of 1 x 1 cm. At 24 hours after the irradiation, each irradiated part was macroscopically observed to determine the minimum erythema dose based on the minimum irradiation dose of ultraviolet rays at which erythema was confirmed.

### <Measurement for melanin index>

Melanin index was measured on "CM-700d", a spectrophotometer commercialized by Konica Minolta Holdings, Inc., Tokyo, Japan.

### <Test samples>

Test samples 1 to 3 in the form of a cream were prepared in usual manner by using "HYDROPHILIC OINTMENT WHEY", a hydrophilic ointment of Japanese Pharmacopoeia as a base, commercialized by Merck Pharmaceutical Company, Germany, and incorporating either or both of guanine and adenine, which were both special-reagent-grade specimens commercialized by Sigma-Aldrich Co. LLC., St. Louis, MO, USA., into the above hydrophilic ointment to give the following concentrations. As a control, only the base for cream was used as test sample 4.
Test sample 1: A cream containing 0.1% by weight of guanine and 0.1% by weight of adenine, d.s.b., to the total cream;
Test sample 2: A cream containing 0.1% by weight of guanine, d.s.b., to the total cream;
Test sample 3: A cream containing 0.1% by weight of adenine, d.s.b., to the total cream; and
Test sample 4: Only the base for cream.

### <Test method>

It was confirmed that there was neither spot nor wound in the inside of the left upper arm of each volunteer. Four test parts of each volunteer were irradiated with 1. 5-times of ultraviolet rays (UVB) of the minimum erythema dose on both the day of initiating the test and the next day. Each irradiation part was made to be an area with a range of 1 x 1 cm for each part applied with each test sample. Just after the initial UVB irradiation, any one of test samples 1 to 4 was applied to each test part three times a day (morning, noon, and evening) for every 28 days by taking about 0.3 g of each test sample per dose on a finger tip. In this case, care was taken not to mix one test sample with any of other test samples. During the test period, the test parts applied with the test samples were cared so as not to be washed within 30 min after the applications. During the volunteers' daily life, the test parts and their neighborhoods were cared so as not to be exposed to a relatively strong ultraviolet rays such as sunlight. The tests were conducted in a double blind manner.

### <Evaluation method>

On the 28th day after initiating the ultraviolet ray irradiation, the test parts were observed macroscopically and measured for melanin index to confirm the degree of pigmentation. The data of 10 volunteers were averaged and shown in Table 15. The degrees of pigmentation by macroscopic observation were determined by scoring based on the following five grades, totaling the scores for each test sample, and averaging the total scores: "Apparently abundant (-2)", "Slightly abundant (-1)", "No difference (0)", "Slightly less (1)", and "Apparently less (2)", where the pigmentations in the test parts applied with test sample 1, 2 or 3 were respectively "apparently abundant", "slightly abundant", "not different", "slightly less", and "apparently less" compared to the test part applied with test sample 4. The degrees of pigmentation by macroscopic observation were judged by five judges for each volunteer and averaging thescores. Themelanin index was determined by regarding the measured value for the test parts applied with test sample 4 as 100%, determining relative values for the test parts applied with any of other test samples, and determining each average. The following are meant in this experiment: The higher the scores of macroscopic observation, the higher the pigmentation-inhibitory effect; while the lower the rate (%) of melanin index, the higher the pigmentation-inhibitory (or the melanin-formation-inhibitory) effect.

**Table 14**

| Test sample | Degree of pigmentation | |
|---|---|---|
| | Macroscopic score | Melanin index (%) |
| 1 | 1.5 | 53 |
| 2 | 0 | 99 |
| 3 | 0.6 | 85 |
| 4 | - | 100 |

As shown in Table 14, the part applied with the cream containing guanine and adenine (test sample 1) was distinctly inhibited pigmentation in view of any of the macroscopic score and the melanin index compared to those of the part applied with the base for cream free of the above compounds (test sample 4). The test parts applied with the cream containing guanine (test sample 2) showed no difference in pigmentation compared to the part applied with test sample 4. The part applied with the cream containing adenine (test sample 3) was inhibited in pigmentation compared to the part applied with test sample 4; however the inhibitory degree was far weaker than that of the part applied with the cream containing adenine and guanine (test sample 1). These results are well coincided with the results in Experiments 2, 4 and 5 and indicate that guanine including derivatives thereof can be used as agents for enhancing the skin-whitening action of adenine including derivatives thereof, and that skin-whitening agents with an improved and enhanced melanin-formation-inhibitory effect can be prepared by incorporating thereunto guanine including derivatives thereof and adenine including derivatives thereof. During the period of and after the test, no induction of abnormality such as red flare and blackout was found other than erythema and pigmentation by ultraviolet ray irradiation in the test parts applied with test samples, and no problem was found in the health conditions of the volunteers. Accordingly, it can be speculated that guanine including derivatives thereof and adenine including derivatives thereof have no safety problem even when applied to the skin.

### <Experiment 10: Influence of guanine including derivatives thereof and equols on the melanin formation in the skin>

Since guanine including derivatives thereof were confirmed to enhance the melanin-formation-inhibitory action (or the skin-whitening action) of equols, the melanin-formation-inhibitory action in the skin induced by suntan (or ultraviolet ray irradiation) was evaluated by the same conditions and method as in Experiment 9 except for replacing adenine with equol (Product Code: "26355-54", a mixture of R- and S-equols, commercialized byNacalaiTesque, Inc., Kyoto, Japan) and 10 male and female volunteers (five each), 25 to 50 years old. The result is in Table 15.

**Table 15**

| Test sample | Degree of pigmentation | |
|---|---|---|
| | Macroscopic score | Melanin index (%) |
| 1 | 1.2 | 63 |
| 2 | 0 | 98 |
| 3 | 0.6 | 83 |
| 4 | - | 100 |

As shown in Table 15, the part applied with the cream containing guanine and equol (test sample 1) was distinctly inhibited pigmentation in view of any of the macroscopic score and the melanin index compared to those of the part applied with the base for cream free of the above compounds (test sample 4). The parts applied with the cream containing guanine (test sample 2) showed no difference in pigmentation compared to the part applied with test sample 4. The part applied with the cream containing equol (test sample 3) was inhibited its pigmentation compared to the part applied with test sample 4; however the inhibitory degree was far weaker than that of the part applied with the cream containing adenine and guanine (test sample 1). These results are well coincided with the results in Experiments 6 and 7, and indicate that guanine including derivatives thereof can be used as agents for enhancing the skin-whitening action of equols and also skin-whitening agents with an improved and enhanced melanin-formation-inhibitory effect can be prepared by incorporating thereunto guanine including derivatives thereof and equols. During the period of and after the test, no induction of abnormality such as red flare and blackout was found other than erythema and pigmentation by ultraviolet ray irradiation in the parts applied with test samples, and no problem was found in the health conditions of the volunteers. Accordingly, it can be speculated that guanine including derivatives thereof and equols have no safety problem even when applied to the skin.

The present invention is explained in more detail with reference to the following Examples but never be restricted thereby. The contents of each ingredient and compounds are expressed with "% by weight" unless specified otherwise. The agents for enhancing the effect of skin-whitening ingredients of the present invention disclosed in the following Examples, or the skin-whitening agents containing any of the above agents and a skin-whitening ingredient (s) in combination can be arbitrarily used as external dermatological agents for skin-whitening or the production materials thereof.

### Example 1

### <Agent for enhancing the effect of skin-whitening ingredients>

One part by weight of guanine was dissolved in four parts by weight of refined water and adjusted to pH 6.8 by admixing with a pH-controlling agent to obtain a liquid agent for enhancing the effect of skin-whitening ingredients. Since the product has an action of enhancing the skin-whitening action inherent to skin-whitening ingredients, particularly, adenine including derivatives thereof and equols, a skin-whitening agent with a satisfactorily skin-whitening effect can be prepared by using adenine including derivatives thereof and/or equols in combination. The product can be also incorporated into external dermatological agents incorporated with adenosines and/or equols as an agent for enhancing the effect of skin-whitening ingredients.

### Example 2

### <Agent for enhancing the effect of skin-whitening ingredients>

One part by weight of guanosine was dissolved in four parts by weight of refined water, adjusted to pH 7.0 by admixing with a pH-controlling agent, spray-dried, and granulated into a granular agent for enhancing the effect of skin-whitening ingredients. Since the product has an action of enhancing the skin-whitening action inherent to skin-whitening ingredients, particularly, adenine including derivatives thereof and equols, a skin-whitening agent with a satisfactorily skin-whitening effect can be prepared by using adenine including derivatives thereof and/or equols in combination. The product can be also incorporated into external dermatological agents incorporated with adenosines and/or equols as an agent for enhancing the effect of skin-whitening ingredients.

### Example 3

### <Agent for enhancing the effect of skin-whitening ingredients>

One part by weight of guanosine and one part by weight of cyclonigerosylnigelose (cyclictetrassacharide) were mixed to homogeneity by stirring to obtain a powdered agent for enhancing the effect of skin-whitening ingredients. Since the product has an action of enhancing the skin-whitening action inherent to skin-whitening ingredients, particularly, adenine including derivatives thereof and equols, a skin-whitening agent with a satisfactorily skin-whitening effect can be prepared by using adenine including derivatives thereof and/or equols in combination. The product can be also incorporated into external dermatological agents with adenosines and/or equols as an agent for enhancing the effect of skin-whitening ingredients.

### Example 4

### <Agent for enhancing the effect of skin-whitening ingredients>

One part by weight of glucosylguanosine used in Experiment 3 and one part by weight of glucosylhesperidin commercialized by Hayashibara Biochemical Laboratories, Inc., Okayama, Japan, were mixed to homogeneity by stirring to obtain a powdered agent for enhancing the effect of skin-whitening ingredients. Since the product has an action of enhancing the skin-whitening action inherent to skin-whitening ingredients, particularly, adenine including derivatives thereof and equols, a skin-whitening agent with a satisfactorily skin-whitening effect can be prepared by using adenine including derivatives thereof and/or equols in combination. The product can be also incorporated into external dermatological agents incorporated with adenosines and/or equols as an agent for enhancing the effect of skin-whitening ingredients.

### Example 5

### <Agent for enhancing the effect of skin-whitening ingredients>

To eight parts by weight of refined water were added one part by weight of glucosylguanosine prepared by the method disclosed in Experiment 3, one part by weight of "TORNARE", a product name of a saccharide composition containing a saccharide derivative of α,α-trehalose commercialized by Hayashibara Biochemical Laboratories, Inc., Okayama, Japan, were mixed to homogeneity by stirring to obtain a liquid agent for enhancing the effect of skin-whitening ingredients. Since the product has an action of enhancing the skin-whitening action inherent to skin-whitening ingredients, particularly, adenine including derivatives thereof and equols, a skin-whitening agent with a satisfactorily skin-whitening effect can be prepared by using adenine including derivatives thereof and/or equols in combination. The product can be also incorporated into external dermatological agents incorporated with adenosines and/or equols as an agent for enhancing the effect of skin-whitening ingredients.

### Example 6

### <Skin-whitening agent>

One part by weight of guanosine and one part by weight of adenine or equol as a skin-whitening ingredient were mixed to homogeneity by stirring to obtain a skin-whitening agent. The product can be also used as an external dermatological agent because the skin-whitening action of adenine or equol is improved and enhanced by guanosine, or it can be also advantageously incorporated into external dermatological agents as a skin-whitening agent therefor.

### Example 7

### <Skin-whitening agent>

Two parts by weight of guanosine 5'-monophosphate and one part by weight of adenosine as a skin-whitening ingredient or one part by weight of S-equol prepared by the method disclosed in Experiment 6 were mixed to produce a skin-whitening agent. The product can be also used as an external dermatological agent because the skin-whitening action of adenosine or S-equol is improved and enhanced by guanosine 5'-monophosphate, or it can be also advantageously incorporated into external dermatological agents as a skin-whitening agent.

### Example 8

### <Skin-whitening agent>

Twopartsbyweightof anyoneof guanosine 5' -monophosphate, guanosine 5'-diphosphate, and guanosine 5'-triphosphate, and one part by weight of any one of adenosine 5'-monophosphate, adenosine 5' -diphosphate, and adenosine 5' -triphosphate were mixed to produce a skin-whitening agent. The product can be also used as an external dermatological agent because the skin-whitening action of any of adenosine phosphates is improved and enhanced by any of guanosine phosphates, or it can be also advantageously incorporated into external dermatological agents as a skin-whitening agent.

### Example 9

### <Skin-whitening agent>

One part by weight of glucosylguanosine prepared by the method disclosed in Experiment 3 and two parts by weight of glucosyladenosine prepared by the method disclosed in Experiment 6 were mixed to produce a skin-whitening agent. The product can be also used as an external dermatological agent because the skin-whitening action of glucosylequol is improved and enhanced by glucosylguanosine, or it can be also advantageously incorporated into external dermatological agents as a skin-whitening agent.

### Example 10

### <Skin-whitening agent>

Four parts by weight of an agent for enhancing the effect of skin-whitening ingredients prepared by the method disclosed in Example 3 and one part by weight of glucosyladenosine as a skin-whitening ingredient prepared by the method disclosed in Experiment 3 or one part by weight of glucosylequol prepared by the method disclosed in Experiment 6 were mixed to produce a skin-whitening agent. The product can be also used as an external dermatological agent because the skin-whitening action of glucosyladenosine or glucosylequol is improved and enhanced by glucosylguanosine, or it can be also advantageously incorporated into external dermatological agents as a skin-whitening agent.

### Example 11

### <Skin-whitening agent>

Four parts by weight of guanosine, one part by weight of glucosyladenosine prepared by the method disclosed in Experiment 3, one part by weight of glucosyl-S-equol prepared by the method disclosed in Experiment 6, and one part by weight of "AA2G", a product name of an ascorbic acid 2-glucoside product commercialized by Hayashibara Biochemical Laboratories, Inc., Okayama, Japan, were mixed to produce a skin-whitening agent. The product can be also used as an external dermatological agent because the skin-whitening actions of glucosyladenosine and glucosyl-S-equol are improved and enhanced by guanosine, or it can be also advantageously incorporated into external dermatological agents as a skin-whitening agent.

### Example 12

### <Cosmetic lotion>

A cosmetic lotion was prepared in usual manner by using the following formulation:

| (Formulation) | (%) |
|---|---|
| Squalane | 8 |
| Polyoxyethylenesorbit tetraoleate | 0.3 |
| Sorbit | 30 |
| "TORNARE", a product name of a saccharide derivative of α,α-trehalose commercialized by Hayashibara Biochemical Laboratories, Inc., Okayama, Japan | 5 |
| Pullulan | 0.001 |
| Ethyl alcohol | 1 |
| Any one of agents for enhancing the effect of skin-whitening ingredients prepared by the methods in Examples 1 to 5 | 1 |
| Adenosine or equol | 1 |
| L-Ascorbic acid-2-glucoside | 2 |
| Astringent (calamine) | 0.1 |
| Antiseptic | q.s. |
| Flavor | q.s. |
| Refined water in an amount sufficient to bring the volume up to 100%. | |

Since the product contains the agent for enhancing the effect of skin-whitening ingredients of the present invention, adenosine or equol as a skin-whitening ingredient, and L-ascorbic acid 2-glucoside, it is a cosmetic lotion having an improved skin-whitening effect of whitening the skin by applying to the skin.

### Example 13

### <Milky lotion>

A milky lotion was prepared in usual manner by using the following formulation:

| (Formulation) | (%) |
|---|---|
| Polyoxyethylenesorbitan monostearate (20 E.O.) | 1 |
| Polyoxyethylenesorbit tetrastearate (60 E.O.) | 0.5 |
| Glyceryl monostearate | 1 |
| Stearic acid | 0.5 |
| Behenyl alcohol | 0.5 |
| Squalane | 8 |
| Any one of skin-whitening agents prepared by the methods in Examples 6 to 11 | 2 |
| Arbutin | 0.2 |
| Antiseptic | 0.1 |
| Carboxyvinylpolymer | 0.1 |
| Sodium hydroxide | 0.05 |
| Ethanol | 5 |
| Flavor | q.s. |
| Refined water in an amount sufficient to bring the volume up to 100%. | |

Since the product contains the skin-whitening agent of the present invention and arbutin as a skin-whitening ingredient, it is a milky lotion having an improved skin-whitening effect of whitening the skin by applying to the skin.

### Example 14

### <Cream>

A cream was prepared in usual manner by using the following formulation:

| (Formulation) | (%) |
|---|---|
| Decaglyceryl monomyristate | 3 |
| Purified lanoline | 0.5 |
| 2-Octyldodecanol | 2 |
| Cetyl 2-ethylhexanoate | 3 |
| Hexamethyltetracosane | 5 |
| Methylpolysiloxane | 0.3 |
| Behenylalcohol | 3 |
| Cetanol | 2 |
| Glyceryl monostearyl alcohol | 1 |
| Cetyl palmitate | 2 |
| Glyceryl monostearate | 2.3 |
| Any one of the skin-whitening agents prepared by the methods in Examples 6 to 11 | 1 |
| Tranexamic acid | 0.5 |
| 1,3-Butylene glycol | 5 |
| 1,2-Pentane diol | 3.5 |
| Glycerin | 6 |
| Glucosylrutin | 0.05 |
| 1% Citric acid | 1 |
| Refined water in an amount sufficient to bring the volume up to 100%. | |

Since the product contains the skin-whitening agent of the present invention and tranexamic acid as a skin-whitening ingredient, it is a cream having an improved skin-whitening effect of whitening the skin by applying to the skin.

### Industrial Applicability

As described above, the agent for enhancing the effect of skin-whitening ingredients containing guanine including derivatives thereof as an effective ingredient(s) can be advantageously used as an agent for enhancing the effect of skin-whitening ingredients that effectively enhances the skin-whitening action of skin-whitening ingredients, particularly, adenosine including derivatives thereof and/or equols; and the skin-whitening agent of the present invention, which contains both the agent for enhancing the effect of skin-whitening ingredients containing guanine including derivatives thereof as an effective ingredient(s) and a skin-whitening ingredient, particularly, adenosine including derivatives thereof and/or equols, can be used as a skin-whitening agent with an improved and enhanced melanin-formation-inhibitory action, and any of the above agents can be advantageously used in the fields of producing external dermatological agents such as cosmetics, pharmaceuticals, quasi-drugs, miscellaneous goods, and chemicals. The present invention, which has such an outstanding function and effect, is a significantly meaningful invention that greatly contributes to the art.

## Claims

1. An agent for enhancing the effect of skin-whitening ingredients, which comprises one or more members selected from the group consisting of guanine including derivatives thereof as an effective ingredient(s).

2. The agent of claim 1, wherein said guanine including derivatives thereof is one or more members selected from the group consisting of guanine, guanosine, guanosine monophosphate, guanosine diphosphate, guanosine triphosphate, and glucosylguanosine.

3. The agent of claim 1 or 2, which enhances the skin-whitening action of adenine including derivatives thereof and/or equol including derivatives thereof.

4. The agent of claim 3, wherein said adenine including derivatives thereof is one or more members selected from the group consisting of adenine, adenosine, adenosine monophosphate, adenosine diphosphate, adenosine triphosphate, and glucosyladenosine.

5. The agent of any one of claims 1 to 3, wherein said equol including derivatives thereof is equol and/or glycosylequol.

6. A skin-whitening agent, which comprises any one of the agents of claims 1 to 4 and adenine including derivatives thereof, wherein the molar ratio of said guanine including derivatives thereof and said adenine including derivatives thereof is 1:199 to 99:1.

7. A skin-whitening agent, which comprises any one of the agents of claims 1 to 3 and 5 and equol including derivatives thereof, wherein the molar ratio of said guanine including derivatives thereof and said equol including derivatives thereof is 1:199 to 99:1.

8. The skin-whitening agent of claim 6 or 7, which further contains one or more skin-whitening ingredients other than said adenine including derivatives thereof and said equol including derivatives thereof.

9. An external dermatological agent, which comprises said skin-whitening agent of any one of claims 6 to 8 in an amount of 0.001 to 5% by weight in terms of guanine.
